Europäisches Patentamt

**European Patent Office**

**Office européen des brevets**

(19)

(11) Publication number: **0 024 642**
**A1**

(12) # EUROPEAN PATENT APPLICATION

(21) Application number: **80104813.3**

(22) Date of filing: **14.08.80**

(51) Int. Cl.³: **C 07 J 9/00**

(30) Priority: **20.08.79 US 68033**

(43) Date of publication of application:
**11.03.81 Bulletin 81/10**

(84) Designated Contracting States:
**DE FR GB NL**

(71) Applicant: **EASTMAN KODAK COMPANY**
**343 State Street**
**Rochester, New York 14650(US)**

(72) Inventor: **Foster, Charles Howard**
**P.O. Box 511**
**Kingsport Tennessee(US)**

(74) Representative: **Brandes, Jürgen, Dr.**
**Dipl.-Chem. et al,**
**Patentanwälte H. Bartels, Dipl.-Chem. Dr. Brandes**
**Dr.-Ing. Held, Dipl.-Phys. Wolff Thierschstrasse 8**
**D-8000 München 22(DE)**

(54) **Separation of stigmasterol from phytosterol.**

(57) This invention relates to the separation of stigmasterol from phytosterols containing stigmasterol, sitosterol and campesterol. Phytosterols are reacted to form the corresponding Δ4-3-keto derivatives of the phytosterols. The resulting phytosterol derivatives are ozonized, and the ozonide is reduced to form 3-ketodinor-4-cholen-22-aldehyde which can be separated from the other phytosterol derivatives.

EP 0 024 642 A1

-1-

## SEPARATION OF STIGMASTEROL FROM PHYTOSTEROL

This invention relates to the separation of stigmasterol from phytosterols containing stigmasterol, sitosterol and campesterol.

The naturally occurring phytosterol components of vegetable oils are composed of mixtures of phytosterols which have similar chemical and physical properties, making separation of the phytosterols into individual components difficult. For example, oils such as soybean oil contain phytosterols which are mixtures of about 25 percent stigmasterol and about 75 percent sitosterol and campesterol. The stigmasterol is extremely difficult to separate from the sitosterol because the two compounds differ structurally by a single double bond in the aliphatic side chain. The separation of stigmasterol from sitosterol and the other phytosterols such as campesterol is important because stigmasterol can be used in the preparation of pharmaceuticals such as progesterone.

Prior to this invention, one method for the separation of stigmasterol from phytosterol mixtures was carried out by the Winders and Hauth bromination method whereby a mixture of phytosterol acetates is brominated, and the relatively insoluble stigmasterol acetate tetrabromide is crystallized. However, this bromination procedure is unsatisfactory for large scale commercial processes for the separation of stigmasterol because it is a relatively expensive process, and the yields are reported to be low.

Other processes, such as extraction or leaching processes, have also been developed and used in commercial processes for separation of stigmasterol. These extraction or leaching processes involve countercurrent extraction or leaching employing large amounts of different solvents. Extensive processing steps, expensive equipment and substantial amounts of labor and energy to isolate stigmasterol are required. The process of this invention is a relatively simple, inexpensive and less energy and labor consuming process useful commercially to separate stigmasterol from other phytosterols.

In accordance with this invention a mixture of phytosterols containing stigmasterol, sitosterol and campesterol is

    a)    converted to the $\Delta^4$-3-keto derivatives of the phytosterols,

    b)    the $\Delta^4$-3-keto derivatives are ozonized,

    c)    the ozonide is reduced to form a mixture of 3-ketodinor-4-cholen-22-aldehyde and the $\Delta^4$-3-keto derivatives of sitosterol and campesterol and

    d)    the 3-ketodinor-4-cholen-22-aldehyde is separated from the mixture.

The specific example provides a preferred procedure for carrying out the process of this invention. The mixture of phytosterols can be converted to the $\Delta^4$-3-keto derivatives by the catalytic oxidation of the $\Delta^5$-sterols to ketones with a simultaneous shift of the $\Delta^5$ double bond to the $\Delta^4$ position in conjugation with the carbonyl group. This transformation is usually carried out by an Oppenauer oxidation such as Recl. Trav. Chim. Pays-Bas., 56, 137 (1937). Raney nickel has also been used for this reaction, either in presence of excess catalyst, as for example, Chakravarti, Chakravarti, and Metra, Nature, 193, 1071 (1962) or in the presence of a hydrogen acceptor, as for example, E. C. Kleiderer, and E. C. Kornfeld, J. Org. Chem., 13, 455 (1948) and Kleiderer, Rice, Conquest and Williams, U.S. Dept. of Commerce, Office of the Publication board, Report PB 981, 1945.

The mixed $\Delta^4$-3-keto derivatives of phytosterol are then treated with ozone or a mixture of ozone and oxygen at a temperature of -80°C. to 0°C. in a suitable solvent such as a 3:1 to 1:3 methylene dichloride/methyl alcohol solvent. The ozonide is reduced with a reducing agent such as trimethyl phosphite and the mixture can be washed with aqueous sodium sulfite, then washed with water, and the product subsequently dried over sodium sulfate. The solvent is then evaporated to provide a yellow oil containing a mixture of 3-ketodinor-4-cholen-22-aldehyde and the $\Delta^4$-3-keto derivatives of sitosterol and campesterol. The 3-ketodinor-4-cholen-22-aldehyde can be separated from this mixture by chromatography or by preparing the bisulfite adduct. The 3-ketodinor-4-cholen-22-aldehyde can be used as a starting material to prepare other steroids such as progesterone.

The ozonide can be reduced by other reducing means in place of the trimethyl phosphite. For example, alternate reducing agents such as the combination of zinc and acetic acid, sodium bisulfite, dimethyl sulfide, formaldehyde and the like reducing agents can also be used.

EXAMPLE

Preparation of $\Delta^4$-3-Keto Soy Steroids

To 150 g. of mixed soy sterols in a 2-liter flask was added 1030 g. cyclohexanone and 100 ml of toluene. The solution was heated to reflux to remove a small amount of water and aluminum isopropoxide was added, heated to reflux for 15 minutes and then distilled with steam to remove excess cyclohexanone. The residue was diluted with water and acidified with concentrated hydrochloric acid and extracted with hexane. The hexane solution was washed three times with water and dried with anhydrous sodium sulfate. The hexane solution was put through a column packed with commercial Doucil (a sodium alumino silicate base exchange material). Some of the $\Delta^4$-3-keto steroids were eluted with hexane in the first fraction with cyclohexylidine cyclohexanone. Elution with 3% acetone in hexane gave 73.3 g. of $\Delta^4$-3-keto soy steroids.

The mixture of $\Delta^4$-3-keto soy steroids was then treated with ozone to prepare 3-ketodinor-4-cholen-22-aldehyde. The ozonization was carried out by a stream of ozone/oxygen being passed through a solution of $\Delta^4$-3-keto soy steroids in 280 ml of methylene chloride containing 3.5 ml. of pyridine at -65 to -75°C. until all of the 4, 22-stigmastadiene-3-one had been reacted. The mixture was flushed with nitrogen to remove excess ozone and then treated with 12.5 g. zinc dust and 25 ml of glacial acetic acid. After stirring 2 hours at ambient temperature the excess zinc dust was filtered and rinsed with hexane. The filtrate was washed with water and dried with anhydrous $Na_2SO_4$.

To a column packed with commercial Doucil was added 28 g. of the oil recovered above in 100 ml of hexane. Elution of the unreacted $\Delta^4$-3-keto steroids with heptane (1250 ml) and with 3% acetone in hexane followed by elution with 30% acetone in hexane gave 9.38 g. of oil which was essentially pure 3-keto-dinor-4-cholen aldehyde.

The 3-keto-dinor-4-cholen-22-aldehyde can also be separated from the $\Delta^4$-3-keto steroids of sitosterol and campesterol by formation of the bisulfite adduct. The ozonolysis product ($\Delta^4$-3-keto steroids) (29.0 g.) was mixed with ethyl alcohol (200 ml.) A saturated aqueous sodium bisulfite solution (250 ml) was added and the mixture was refluxed for 1 hour. The mixture was cooled to 10°C. and a white solid was isolated by filtration. The solid was washed with toluene. Evaporation of toluene gave 19.0 g. of oil which was unreacted $\Delta^4$-3-keto steroids. The original ethyl alcohol/water/sodium bisulfite filtrate was further extracted with toluene. Evaporation of the toluene layer gave 3-keto-dinor-4-cholen-22-aldehyde as a viscous oil (7.5 g.).

Alternatively, the ozonolysis product ($\Delta^4$-3-keto steroids) (17.0 g.), dissolved in ethanol (150 ml) was treated with a solution prepared from 12 ml of ethanol and 48 ml saturated aqueous sodium bisulfite solution. The mixture was heated on a steam bath for 1 to 5 hours, then allowed to cool to room temperature. The precipitate obtained was filtered off and treated with 100 ml of 5% sodium hydroxide. Filtration of this mixture gave 3-keto-dinor-4-cholen-22-aldehyde in 69% yield.

0024642

-6-

<u>Claims:</u>

1. The process for separating stigmasterol from a mixture of phytosterols containing stigmasterol, sitosterol and campesterol characterized by

a) converting the phytosterols to the $\Delta^4$-3-keto derivatives of the phytosterols,

b) ozonizing the $\Delta^4$-3-keto derivatives of the phytosterols,

c) reducing the ozonide to form a mixture of 3-ketodinor-4-cholen-22-aldehyde and the $\Delta^4$-3-keto derivatives of sitosterol and camposterol, and

d) separating 3-ketodinor-4-cholen-22-aldehyde from said mixture.

2. The process according to Claim 1, wherein the 3-ketodinor-4-cholen-22-aldehyde is separated by chromatography.

3. The process according to Claim 1, wherein the 3-ketodinor-4-cholen-22-aldehyde is separated by forming a bisulfite adduct.

European Patent
Office

**EUROPEAN SEARCH REPORT**

Application number

EP 80 10 4813

| Category | Citation of document with indication, where appropriate, of relevant passages | Relevant to claim |
|---|---|---|
| X | US - A - 2 782 192 (RAYMOND L. PEDERSON) <br> * Example 4, claims 7-9 * | 1,2 |
| E | EP - A - 0 016 380 (EASTMAN KODAK) <br> * Claims 1-3 * | 1-3 |

**DOCUMENTS CONSIDERED TO BE RELEVANT**

CLASSIFICATION OF THE
APPLICATION (Int. Cl.³)

C 07 J 9/00

TECHNICAL FIELDS
SEARCHED (Int. Cl.³)

C 07 J 9/00

CATEGORY OF
CITED DOCUMENTS

X: particularly relevant
A: technological background
O: non-written disclosure
P: intermediate document
T: theory or principle underlying
the invention
E: conflicting application
D: document cited in the
application
L: citation for other reasons

&: member of the same patent
family,
corresponding document

The present search report has been drawn up for all claims

| Place of search | Date of completion of the search | Examiner |
|---|---|---|
| The Hague | 20-10-1980 | HENRY |

EPO Form 1503.1  06.78